Europäisches Patentamt

European Patent Office

Office européen des brevets

(19)

(11) Publication number: **0 491 042 A1**

(12)

## EUROPEAN PATENT APPLICATION
### published in accordance with Art. 158(3) EPC

(21) Application number: 90913256.5

(22) Date of filing: **06.09.90**

(86) International application number:
**PCT/JP90/01142**

(87) International publication number:
**WO 91/03567 (21.03.91 91/07)**

(51) Int. Cl.5: **C12P 19/60**, C12P 1/00,
//C12N5/04,(C12P19/60,
C12R1:91),(C12P1/00,
C12R1:91)

(30) Priority: **08.09.89 JP 234259/89**

(43) Date of publication of application:
**24.06.92 Bulletin 92/26**

(84) Designated Contracting States:
**AT BE CH DE DK ES FR GB IT LI LU NL SE**

(71) Applicant: **NIPPON SHINYAKU CO., LTD.
14, Kisshoinnishinoshomonguchicho
Minami-ku Kyoto-shi Kyoto 601(JP)**

(72) Inventor: **HINA, Yasuhiko
14-19, Osumigaoka 4-chome, Tanabecho
Tsuzuki-gun, Kyoto 610-03(JP)**
Inventor: **AKITA, Toru
10-205, Nagitsuji, Nakazaikecho,
Yamashina-ku
Kyoto-shi, Kyoto 607(JP)**
Inventor: **NISHI, Toyoyuki
35, Nishimachi
Kameoka-shi, Kyoto 621(JP)**

(74) Representative: **Kügele, Bernhard et al
c/o NOVAPAT-CABINET CHEREAU 9, Rue du
Valais
CH-1202 Genève(CH)**

(54) **METHOD OF PRODUCING BEET RED.**

(57) A method of producing beet red, a food dye obtained from a beet and used widely, by the callus tissue culture technique efficiently, which is characterized by (1) using cells of any plant of the genus Beta, (2) applying liquid culture, and (3) culturing under aerobic conditions. The obtained dye has various advantages, for example, that it has a good color tone and a color value, and can be prepared aseptically so that it can be served as a food as such without the necessity for sterilization.

EP 0 491 042 A1

FIELD OF THE INVENTION

The present invention relates to a process for preparing a food dye called beet red.

Beet red is a red food colorant containing as a main component betanin of betacyanins which is extracted from the root of table beet (Beta vulgaris L. var. rubra) cultivated in Europe, etc.

Because of its good hue, beet red has been widely used for cakes such as ice cream, etc., beverage, Japanese unbaked cakes, dairy products; processed marine foods such as salted pollack roe, salted fish guts, etc.; processed livestock foods such as sausage, humburger, etc. Beet red is a natural product and hence, would be much in demand also in the future, along with the trend of natural food taste.

BACKGROUND OF THE INVENTION

Beet red has been hitherto prepared by collecting the root of raw plants and extracting from the root. Accordingly, its yield greatly varied depending upon natural environments. Also depending on lot, its quality widely varied.

For the purpose of obtaining beet red with a constant quality in large quantities, a method for plant tissue culture was proposed. With respect to the technique for producing betacyanins by plant tissue culture, there are the following reports.

Berlin et al. makes a report on liquid culture of a plant belonging to the genus Chenopodium - (Chenopodium rubrum) [Jochen Berlin et al., Plant Cell Tissue Organ Culture, 5, 163-174].

Arai et al. report on the betacyanin synthesis system of a plant belonging to the genus Chenopodium - (Chenopodium album) on solid medium in the callus culture system.

Bohm et al. report on the synthesis of betacyanins of Portulaca grangiflora on solid medium in the callus culture system [Winnie Schroder and Hartmut Bohm, Plant Cell Reports, 3, 14-17].

Sakuta et al. report on a mechanism of controlling betacyanin formation using the liquid culture cell system of Phytolacca americana [Masaki Sakuta et al., Physiologia Plantarum, 71, 455-458, and so on].

However, raw materials used in these reports are not generally used for food and the production efficiency is also poor so that the method is not practical.

Constabel [Friedrich Constabel, Naturwissenschaften, 7, 175-176], Bornman et al. [Carin Jarl and Chris H. Bornman, Hereditas, 105, 55-59] and Girod et al. [P.A. Girod and J.P. Zryd, Plant Cell Reports, 6, 27-30] report on callus cultivation of a plant belonging to the genus Beta (Beta vulgaris).

These reports deal with utilization of tissue culture and are different in this regard from the other reports. However, a solid medium is used so that the productivity is poor.

DISCLOSURE OF THE INVENTION

In order to overcome the actual situation described above, the present inventors have made various investigations. Therefore, an object of the present invention is to establish the means for supplying beet red as a natural dye stably in a large quantity.

The present invention comprises steps of culturing cells for preparing beet red and collecting beet red therefrom.

The gist of the present invention lies in the following points. That is, upon plant tissue culture, the present invention requires that:

(1) cells of a plant belonging to the genus Beta as cells to be cultured;

(2) the cells are subjected to liquid culture;

(3) the cells are cultured under aerobic conditions; and at the same time, meets 0 or at least one of the requirements 1. through 9. described below:

1. auxin is added as a component in liquid medium;

2. cytokinin is added as a component in liquid medium;

3. the cells are cultured under light conditions;

4. a part of the medium already provided for culture is added to a fresh liquid medium;

5. ammonium ions ($NH_4^+$) are present in the medium in a far lower concentration than in LS medium;

6. nitrate ions ($NO_3^-$) are present in the medium in a far lower concentration than in LS medium;

7. potassium ions ($K^+$) are present in the medium in a far lower concentration than in LS medium;

8. borate ions ($BO_3^{3-}$) are present in the medium in a far lower concentration than in LS medium; and,

9. iron ions ($Fe^{2+}$) are present in the medium in a far lower concentration than in LS medium.

BEST MODE FOR PRACTICING THE INVENTION

As th plant belonging to the genus Beta used in the present invention, the plants belonging to the genus Beta of all kinds which are used for food may be employed.

Examples of such plants include table beet(Beta Vulgaris var, rubra),chard(Beta vulgaris var. cicla),sugar beet(Beta vulgaris var. saccharifera),fodder beet(Beta vulgaris var. rapa),etc.

In the present invention, any part of the plant body may be used and is exemplified by a root, a stalk, a seed, etc.

In the present invention, it is necessary to aseptically prepare a plant sample for callus induction.

For example, where a seed is used in the present invention, seeds are treated like sterilizatns, etc. in a conventional manner and then seeded on a solid medium for germination. The thus obtained sample is used.

In the present invention, a part of a plant grown in a field may also be sterilized to obtain an aseptic sample.

The thus obtained sample is cut into a suitable size and placed on a solid medium for callus induction to induce and grow callus.

The thus induced and grown callus is cut into pieces of a suitable size and subcultured on a solid medium followed by culture. By repeating the procedure of subculture, a uniform callus can be obtained.

The number of such subculture can be appropriately increased or decreased but in general, it is sufficient to perform twice to several times. It is generally sufficient to perform one subculture every a few weeks.

In the medium for subculture described above, auxins such as 2,4-dichlorophenoxyacetic acid (hereinafter abbreviated as "2,4-D") or the like may be incorporated, for the purpose of improving its growth.

As auxins, IAA (indoleacetic acid), NAA (1-naphthylacetic acid), etc. which are conventionally used may also be used, in instead of 2,4-D.

Repeatedly subcultured callus to a sufficient extent may be then subjected to suspension cultre in a liquid medium. Shaking may be carried out under conditions as in ordinary tissue culture but it is sufficient to shake with an amplitude of about 70 mm, e.g., at 90 rpm.

culture may be conducted under dark conditions where a light is shielded but in general, it is preferred to perform culture under light conditions, e.g., under a light of about 3,000 lux. By this procedure, callus is converted into a suspension of a mass of small cells while growing and proliferating.

The suspended cells are further repeatedly subjected to culture in a liquid medium thereby to effect subculture.

In this suspension culture, for example, a flask, may be used as a container. The opening of a flask, etc., may be covered with a filter such as an aluminum foil generally used for plant tissue culture to prevent contamination. The culture may also be performed under aerobic conditions, using a filter having a high oxygen permeability such as a PF microfilter, etc. instead of the aluminum foil. It is revealed that in the present invention, culture under aerobic conditions is suited for the purpose.

The present invention may be practiced in a laboratory scale using, e.g., a flask but may also be practiced in a larger, industrial scale using, e.g., a jar fermenter of 30 liters. Also in this case, it is revealed that the yield of the desired pigment is always preferable.

The thus subcultured cell suspensions as described above are transplanted into a liquid medium for producing the dye, cultured and proliferated to produce raw cells for preparing beet red.

The cells are isolated from the culture broth by means of centrifugation, etc. and repeatedly washed with water to obtain the cells.

The obtained cells are pressed so that the cell contents are obtained as a mixture. The mixture is centrifuged to remove insoluble matters and the supernatant is freeze dried to obtain the beet red in accordance with the present invention.

The thus obtained beet red in accordance with the present invention can be pulverized without adding any excipient thereto.

The beet red has the maximum absorption at 535 nm in its visible light absorption spectrum which is the same as that of known commercially available beet red. This absorption corresponds to the maximum absorption zone of betanin, indicating that the beet red in accordance with the present invention is a colorant abundant in betanin.

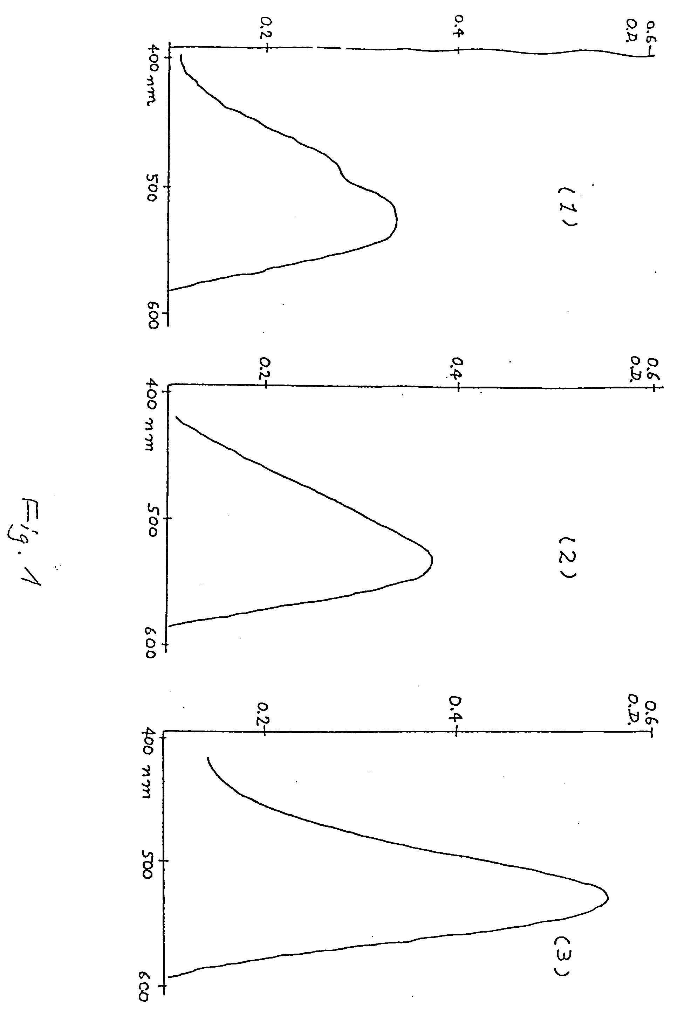

The Absorption spectra of the beet red prepared by the present invention, commercially available beet red and the root of table beet were determined, respectively, following the method for measuring a pigment content, whereby spectra as shown in Fig. 1 were obtained. Fig. 1 indicates that the spectrum of beet red derived from culture cells has the same absorption maxim at 535 nm as in the spectrum of commercially available beet red or table beet. Ratio in absorbance between the maximum absorption of betanin at 535 nm

3

and the maximum absorption of betaxanthin at 480 nm is shown in the following table.

The present invention is characterized in that the beet red prepared by the process of the present invention has a more blue color than the beet red prepared in a conventional manner and hence, can provide a preferable color when added to food.

| Comparison in Absorption Spectrum | | | |
|---|---|---|---|
| | (1) 480 nm | (2) 535 nm | (1)/(2) |
| Root of table beet | 0.256 | 0.336 | 0.76 |
| Commercially available beet red | 0.252 | 0.376 | 0.67 |
| Beet red of this invention | 0.268 | 0.560 | 0.48 |

The present invention is also characterized in that the beet red prepared by the process of the present invention can be supplied aseptically so that the beet red can be used for food as it is, without performing any complicated operations such as sterilization and the like. The present invention is further characterized in that the beet red prepared by the process of the present invention has a high color value and therefore is applicable to food having a deep color.

[Examples]

Hereinafter the present invention will be described in more detail by referring to the examples.

Example

(1) Callus induction and subculture

Seeds of table beet produced in USA (Beta vulgaris L. var. rubra cv. Extra Early Flat Egyptian) (acquired from Fujita Nursery in November, 1985) were treated with 70% ethanol for 10 seconds and 1% antiformine for 5 minutes for sterilization and then aseptically seeded on Linsmaier and Skoog medium (LS medium) having the following composition and supplemented with 3% sucrose and 1% agar.

Composition of LS medium

$KNO_3$ 1,900 mg/l, $NH_4NO_3$ 1,650 mg/l, $MgSO_4 \cdot 7H_2O$ 370 mg/l, $CaCl_2 \cdot 2H_2O$ 440 mg/l, $KH_2PO_4$ 170 mg/l, $Na_2$-EDTA 37.3 mg/l, $FeSO_4 \cdot 7H_2O$ 27.8 mg/l, $MnSO_4 \cdot 4H_2O$ 22.3 mg/l, $ZnSO_4 \cdot 7H_2O$ 8.6 mg/l, $H_3BO_3$ 6.2 mg/l, KI 0.83 mg/l, $Na_2MoO_4 \cdot 2H_2O$ 0.25 mg/l, $CuSO_4 \cdot 5H_2O$ 0.025 mg/l, $CoCl_2 \cdot 6H_2O$ 0.025 mg/l, myo-Inositol 100 mg/l,
Thiamine HCl 0.4 mg/l

After seedling in the dark, seeds which grew to about 10 cm at the hypocotyl were used as sample for callus induction. The thus obtained aseptic hypocotyl was cut into segments each having a size of about 3 mm and aseptically placed on LS medium containing $10^{-7}$ M of 2,4-D.

The hypocotyl segment was cultured at 25°C in the dark for 78 days. During the incubation, a purple red callus was induced from the segment and grew.

The callus was cut into a size of about 30 mg and aseptically subcultured on LS medium containing $10^{-7}$ M of 2,4-D, 3% sucrose and 1% agar. This procedure for subculture was repeated 3 times every 4 other weeks to obtain a uniform purple red callus.

(2) Induction and subculture of suspended cells

About 1 g of this callus was aseptically transferred into 50 ml of LS liquid medium containing $3 \times 10^{-7}$ M of 2,4-D and 3% sucrose which was charged in an Erlenmeyer's flask of 100 ml volume, followed by suspension culture at 90 rpm (about 70 mm in reciprocating amplitude). The callus in the liquid medium became a suspended state of small cell masses and grew and proliferated there.

A definite amount (about 100 mg when calculated on dry weight basis) of the suspended cells was aseptically transferred to LS liquid medium having the same composition and subcultured every 3 other weeks.

(3) Growth test in test medium

Various conditions of test medium were examined below. In each run, growth test was performed as follows unless otherwise indicated.

(1) Determination of the amount of cells obtained:

A definite amount (about 50 mg when calculated on dry weight basis) of the suspended cells was aseptically transferred to 25 ml of test medium charged in an Erlenmeyer's flask of 100 ml volume followed by suspension culture. After incubation for a definite period of time, the cells were filtered and lyophilized. The weight of the dried cells was measured and the yield of cells per flask was determined.

In the suspension culture, a set of aluminum foil in 4 sheets conventionally used for plant tissue culture was used as a filter of the flask opening to prevent contamination, except for special occasion. The incubation was performed by reciprocating suspension culture at 25°C and 90 rpm in an amplitude of 70 mm in the dark.

(2) Determination of pigment content:

The pigment was quantitatively determined by a modification of the method for determination of color value defined in natural food additive standard by the Federation of Japanese Associations on Food Additives. Dry cells were mashed in a mortar to be homogeneous. A definite amount (20 mg) was weighed and extracted with acetic acid/sodium acetate buffer (10 ml), pH 5.4. Absorbance was measured at 535 nm with a spectrophotometer. The betanin content was calculated from the molecular weight (550.47) and molecular extinction coefficient ($\epsilon$ 60,500) of betanin, following the equation below:

$$\text{pigment content (\%)} = A_{535} \times \frac{550.47 \times 10}{60500 \times 20} \times 100$$

$A_{535}$ :   absorbance at 535 nm of wavelength

(3) Amount of pigment yielded:

The amount of pigment yielded per flask was calculated from the amount of cells obtained and the pigment content, per flask, described above.

$$\text{Amount of pigment (mg)} = \frac{\text{Amount of cells (mg)} \times \text{pigment content (\%)}}{100}$$

(1) Run 1

Kind and concentration of auxin:

The suspended cells were transferred to LS liquid medium in which the concentration of sucrose was fixed to 3% and 2,4-D as plant growth regulator auxin was varied over the range of $2 \times 10^{-7}$ to $7 \times 10^{-7}$ M and IAA and NAA was varied over the range of $3 \times 10^{-6}$ to $1 \times 10^{-4}$ M, respectively, and subjected to suspension culture.

The amount of cells obtained, pigment content and amount of the pigment obtained were examined on Day 17 after culture. The results are shown in the following table. The numerical value shows the results of culturing the suspended cells in terms of an exponent when the results under conditions of ordinary subculture (2,4-D, $3 \times 10^{-7}$ M) are made 100.

5

|  |  | Amount of Cell | Pigment Content | Amount of Pigment |
|---|---|---|---|---|
| Z.4D | $2 \times 10^{-7}$ M | 112 | 98 | 110 |
|  | $3 \times 10^{-7}$ M | 100 | 100 | 100 |
|  | $5 \times 10^{-7}$ M | 93 | 91 | 84 |
|  | $7 \times 10^{-7}$ M | 96 | 89 | 85 |
| IAA | $3 \times 10^{-6}$ M | 47 | 37 | 17 |
|  | $1 \times 10^{-5}$ M | 69 | 55 | 38 |
|  | $3 \times 10^{-5}$ M | 96 | 87 | 83 |
|  | $1 \times 10^{-4}$ M | 75 | 80 | 60 |
| NAA | $3 \times 10^{-6}$ M | 53 | 42 | 22 |
|  | $1 \times 10^{-5}$ M | 63 | 68 | 43 |
|  | $3 \times 10^{-5}$ M | 87 | 96 | 83 |
|  | $1 \times 10^{-4}$ M | 83 | 98 | 81 |

The results reveal that the suspended cells grow well and produce a pigment in a concentration of 2,4-D of $2 \times 10^{-7}$ to $7 \times 10^{-7}$ M. The results also reveal that the cells for producing a pigment sufficiently grow with IAA or NAA in a concentration of about $3 \times 10^{-5}$ M.

(2) Run 2

Kind and concentration of sugars as carbon sources:

The suspended cells were transferred to LS liquid medium in which the concentration of 2,4-D was fixed to $3 \times 10^{-7}$ M and the concentration of sugars (sucrose, glucose, maltose) as carbon sources was varied over the range of 2 to 7%, respectively, and subjected to suspension culture.

The amount of cells obtained, pigment content and amount of the dye obtained were examined on Day 17 after culture. The results are shown in the following table. The numerical value shows the results of culturing the suspended cells in terms of an exponent when the results under conditions of ordinary subculture (sucrose, 3%) are made 100.

|  | Amount of Cell | Pigment Content | Amount of Piment |
|---|---|---|---|
| Test 1 — Sucrose 2% | 81 | 81 | 66 |
| Sucrose 3% | 100 | 100 | 100 |
| Sucrose 5% | 111 | 74 | 83 |
| Sucrose 7% | 104 | 64 | 68 |
| Glucose 2% | 75 | 100 | 74 |
| Glucose 3% | 82 | 89 | 74 |
| Glucose 5% | 69 | 73 | 51 |
| Glucose 7% | 49 | 51 | 26 |
| Test 2 — Sucrose 3% | 100 | 100 | 100 |
| Sucrose 4% | 100 | 78 | 78 |
| Sucrose 5% | 122 | 73 | 89 |
| Maltose 3% | 70 | 93 | 65 |
| Maltose 4% | 124 | 78 | 98 |
| Maltose 5% | 125 | 65 | 82 |

6

The results reveal that sucrose or maltose is good for sugars as carbon sources.

With respect to the concentration of sugars, about 5% was good for the amount of cells yielded and about 3% was good for the pigment content.

(3) Run 3

Study on composition of various inorganic salts in LS medium:

The cells were cultured in LS medium in which the ionic concentration of various inorganics was varied, to determine the influence of these ions on cell growth and pigment content.

The suspended cells were transferred to LS liquid medium in which the concentrations of sucrose and 2,4-D were fixed to 3% and $3 \times 10^{-7}$ M, respectively, and the concentration of all constituent ions of $NO_3^-$, $NH_4^+$, $PO_4^{3+}$, $K^+$ ions, was varied and subjected to suspension culture.

The results are shown in terms of an exponent when the results cultured under conditions of ordinary subculture (20.6 mM) are made 100, using ammonium ions as an example.

| $NH_4^+$ Ion Concentration (mM) | Amount of Cell | Pigment Content | Amount of Pigment |
|---|---|---|---|
| 0 | 122 | 123 | 150 |
| 5 | 130 | 124 | 161 |
| 10 | 116 | 126 | 145 |
| 12.5 | 111 | 116 | 129 |
| 15 | 109 | 106 | 115 |
| 17.5 | 104 | 106 | 110 |
| 20.6 | 100 | 100 | 100 |
| 30 | 75 | 58 | 44 |

The results reveal that the amount of cells obtained and the pigment content were increased by about 30% and about 24%, respectively, in the concentration of ammonium ions of 5.0 mM which is about 1/4 of 20.6 mM of the concentration in ordinary LS medium and as the result, the amount of pigment obtained increased by 61%.

Likewise, the respective ions were examined and the results shown in the following table were obtained. The following table shows the results obtained in an improved ionic concentration when the amount of cells obtained, pigment content and amount of pigment obtained were made 100 when the cells were cultured in ordinary ionic concentration of LS medium.

| Ion | Concentration of LS medium — concentration after improvement | Amount of Cell | pigment Content | Amount of pigment |
|---|---|---|---|---|
| $NH_4^+$ | 20.6 mM -- 5.0 mM | 130 | 124 | 171 |
| $NO_3^-$ | 39.4 mM -- 78.8 mM | 140 | 100 | 148 |
| $K^+$ | 20.1 mM -- 11.3 mM | 114 | 143 | 162 |
| $BO_3^{3+}$ | 0.1 mM -- 1.0 mM | 100 | 128 | 129 |
| $Fe^{2+}$ | 0.1 mM -- 1.0 mM | 106 | 196 | 209 |

The results reveal that the productivity can be markedly improved by setting forth:

$NH_4^+$ ions to 5.0 mM which is about 1/4 that of LS medium (medium conventionally used);

$NO_3^-$ ions to 78.8 mM which is about twice that of LS medium;

$K^+$ ions to 11.3 mM which is lower by 8.8 mM than that of LS medium;

$BO_3^{3-}$ ions to 1.0 mM which is a concentration of about 10 times that of LS medium; and

$Fe^{2+}$ ions to 1.0 mM which is a concentration of about 10 times that of LS medium.

(4) Run 4

Concentration of cytokinin:

The suspended cells were transferred to LS liquid medium in which the concentrations of 2,4-D and sugar were fixed to $3 \times 10^{-7}$ M and 5%, and the concentration of benzyladenine (hereafter "BA") as one of plant growth regulator cytokinins was varied over the range of 0 to $7 \times 10^{-7}$ M, respectively, and subjected to suspension culture. The amount of cells obtained, dye cultured in a fresh medium, a part of old medium already used for culture was added in an amount of 10% of the fresh medium. The results shown in the following table were obtained. The amount of cells obtained, pigment content and amount of the pigment obtained were examined on Day 17 after cultivation and the results are shown in terms of an exponent when the results obtained by culturing in a fresh medium alone are made 100. 100.

| Concentration of BA | Amount of Cell | Pigment Content | Amount of Piment |
|---|---|---|---|
| 0 M | 100 | 100 | 100 |
| $2 \times 10^{-7}$ M | 113 | 106 | 120 |
| $3 \times 10^{-7}$ M | 114 | 210 | 239 |
| $7 \times 10^{-7}$ M | 117 | 217 | 250 |

The results reveal that the pigment productivity can be markedly improved by adding BA to the liquid medium. This BA can be replaced by zeatin or kinetin which is a kind of cytokinin and said to exhibit a similar effect.

(5) Run 5

Experiment in which a part of old medium is added to a fresh medium:

The suspended cells were transferred to 50 ml of LS liquid medium containing 3% of sucrose and $3 \times 10^{-7}$ M of 2,4-D, or to a mixture of 45 ml of medium having the same composition and 5 ml of old medium, and subjected to suspension culture.

That is, when the suspended cells were transferred and content and amount of the dye obtained were examined on Day 17 after cultivation. The results are shown in the following table in terms of an exponent when the results cultured when no BA was contained are made 100.

| New Medium : Old Medium | Amount of Cell | Pigment Content | Amount of Pigment |
|---|---|---|---|
| 50 ml: 0 ml | 100 | 100 | 100 |
| 45 ml: 5 ml | 125 | 98 | 122 |

The results reveal that by adding a part of the old medium to the fresh medium in an amount of 10%, the initial growth of the cells was accelerated and the amount of cells increased.

(6) Run 6

Culture under light conditions:

The suspended cells were transferred to LS liquid medium containing 3% of sucrose and $3 \times 10^{-7}$ M of 2,4-D and subjected to suspension culture under light conditions (about 3000 lux) and under dark conditions (0 lux).

The amount of cells obtained, pigment content and amount of the dye obtained were examined on Day 17 after culture and the results are shown in the following table, in terms of an exponent when the results obtained under ordinary dark conditions are made 100.

| Lighting Condition | Amount of Cell | Pigment Content | Amount of Pigment |
|---|---|---|---|
| Under dark conditions | 100 | 100 | 100 |
| Under light conditions | 105 | 163 | 171 |

The results reveal that the light hardly affects cell growth but enhances the amount of pigment and markedly improves pigment productivity.

(7) Run 7

Cultivation in a high oxygen concentration:

The suspended cells were transferred to LS liquid medium containing 3% of sucrose and $3 \times 10^{-7}$ M of 2,4-D and subjected to suspension culture under conditions where the flask opening was covered with a conventional aluminum foil and under conditions where the opening was covered with PF Microfilter (manufactured by Mitomi Industry Co., Ltd.) having a high oxygen permeability. The amount of cells obtained, pigment content and amount of the pigment obtained were examined on Day 17 after culture. The results are shown in the following table, in terms of an exponent when the results obtained with the conventional aluminum foil are made 100.

| Kind of Covering | Amount of Cell | Pigment Content | Amount of Pigment |
|---|---|---|---|
| Aluminum foil | 100 | 100 | 100 |
| PF Microfilter | 96 | 179 | 173 |

The results reveal that the culture using a filter having a high oxygen permeability, that is, aerobic conditions hardly affect cell growth but enhance the amount of pigment so that the pigment productivity is markedly improved.

(8) Run 8

Cultivation in a large culture bottle:

The suspended cells (about 2 g on dry weight basis per liter of medium)were transferred to improved RM medium (later described) (1 to 2 liters) containing 10% of old medium which was charged in a large size culture bottle and cultured for 14 days. During the culture , the medium was stirred at 100 to 300 rpm using a magnet stirrer and air or 99% oxygen was blown onto the surface in an amount of 0.5 liter/min. (bubbled in the liquid in the case of a jar fermenter later described).

The amount of medium, number of stirring and aerial conditions were varied and the results are shown in the following table.

| Condition | Amount of Cell | Pigment Content | Amount of Pigment |
|---|---|---|---|
| Medium, 1 ℓ 100 rpm, air | 6.15 g-DW/l | 0.17 % | 10.5 mg-DW/l |
| Medium, 1 ℓ 100 rpm, oxygen | 15.47 g-DW/l | 0.71 % | 109.8 mg-DW/l |
| Medium, 1 ℓ 300 rpm, oxygen | 24.28 g-DW/l | 0.87 % | 211.2 mg-DW/l |
| Medium, 2 ℓ 200 rpm, oxygen | 31.84 g = DW/l | 0.55 % | 175.1 mg-DW/l |

It is noted that where air was shielded under the conditions described above, the cells did not grow. By changing the gas from air to oxygen, the amount of oxygen dissolved in the culture broth stirred increases. Further by increasing the number of rotation, the amount of oxygen dissolved in the culture broth stirred increases.

In the present invention, it is evident that as the amount of oxygen dissolved in the culture broth increases, the amount of pigment obtained is markedly improved.

(9) Run 9

Cultivation in a jar fermenter of 30 liter volume:

The suspended cells (about 1.13 g on dry weight basis per liter of medium) were transferred to 12 liters of improved RM medium which was charged in a jar fermenter of 30 liter volume, and cultured for 13 days. The results are shown in the following table.

EP 0 491 042 A1

| Amount of Cell | Pigment Content | Amount of Pigment |
|---|---|---|
| 6.19 g-DW/l | 1.1 % | 68.3 mg-DW/l |

The amount of cells obtained indicates an amount (g) per liter of the medium; the pigment content is expressed by % and the amount of pigment obtained is expressed by an amount (mg) per liter of the medium.

The results reveal that the present invention is operable not only on a flask level but also under large scale conditions.

(10) Run 10

culture in improved medium under improved conditions:

Based on the new findings obtained in Run Nos. 5 to 7, the improved culture conditions were set forth. That is, the conditions, the addition of the old medium already used for culture to a fresh medium in an amount of 10%, light conditions and using PF Microfilter having a high oxygen permeability, were added to the ordinary conditions.

The results obtained by growth test in the improved medium under the improved environmental conditions are shown below, as compared to the results obtained in ordinary LS liquid medium for subculture under ordinary culture conditions.

Composition of improved RM medium

$NaNO_3$ 5,424 mg/l, $KNO_3$ 1,011 mg/l, $NH_4NO_3$ 400,mg/l, $MgSO_4 \cdot 7H_2O$ 740 mg/l, $CaCl_2 \cdot 2H_2O$ 440 mg/l, $KH_2PO_4$ 170 mg/l, $FeSO_4$ 278 mg/l, $Na_2$-EDTA 373 mg/l, $MnSO_4 \cdot 4H_2O$ 22.3 mg/l, $ZnSO_4 \cdot 7H_2O$ 8.6 mg/l, $H_3BO_3$ 61.8 mg/l, KI 0.83 mg/l, $Na \cdot MoSO_4 \cdot 2H_2O$ 0.25 mg/l, $CuSO_4 \cdot 5H_2O$ 0.025 mg/l, $CoCl_2 \cdot 6H_2O$ 0.025 mg/l, myo-Inositol 100 mg/l,
Thiamine HCl:0.4 mg/l,
Sucrose: 50.0 g/l, 2.4-D $3 \times 10^{-7}$ M, BA $7 \times 10^{-7}$ M

Table

| | Amount of Cell | Pigment Content | Amount of Piment |
|---|---|---|---|
| Comparative Example | 18.6 g-DW/l | 0.9 % | 164 mg-DW/l |
| RM medium | 29.0 g-DW/l | 1.1 % | 329 mg-DW/l |
| RM medium, under improved conditions | 21.2 g-DW/l | 3.1 % | 652 mg-DW/l |

The amount of cells obtained indicates an amount (g) per liter of the medium; the pigment content is expressed by % and the amount of pigment obtained is expressed by an amount (mg) per liter of the medium.

(4) Preparation of beet red using culture cells as raw materials

The suspended culture cells subcultured were transferred to a medium for producing pigment, cultured and proliferated to obtain cells as raw materials for producing beet red. The cells were isolated from the culture broth by centrifugation and washed with water twice.

By pressing 1300 g of the cells, juice was obtained. The juice was further centrifuged to remove insoluble matters. The juice was freeze dried to obtain beet red derived from culture cells.

The beet red was pulverized without adding any excipient thereto to obtain the product showing a color value of 111 (betanin content, 1.01%).

In conventional beet red, large amounts of hygroscopic solid contents are also extracted simultaneously so that it is necessary to add an excipient to an extent amount, otherwise it is impossible to obtain powdery products. Thus, the products have only a low color value. However, the beet red derived from culture cells provides a high color value, since betanin is produced concentratively in the culture system.

10

INDUSTRIAL APPLICABILITY

The beet red in accordance with the present invention can be used for the same application as in conventional beet red. The beet red in accordance with the present invention is abundant in betanin as a pigment component. Accordingly, the best red in accordance with the present invention is useful as a red colorant.

The beet red in accordance with the present invention has a high color value so that where such a colorant is applied to food, the purpose can be achieved by addition in a smaller amount than in conventional products. Therefore, food having a high commercial value can be prepared.

The beet red in accordance with the present invention can be prepared by plant tissue culture and can thus be provided aseptically. Therefore, the beet red in accordance with the present invention can be used for food as it is, without performing complicated operations such as sterilization, etc.

According to the present invention, beet red having the quality equivalent to or higher than that of conventional beet red can be permanently provided at low costs.

BRIEF DESCRIPTION OF THE DRAWING

Fig. 1 shows absorption spectrum of the beet red prepared by the process of the present invention, wherein (1) denotes the root of table beet, (2) denotes commercially available beet red and (3) denotes the beet red prepared by the process in accordance with the present invention. The vertical axis indicates absorbance and the abscissa indicates wavelength (nm).

**Claims**

1. A process for preparing beet red utilizing plant tissue culture which comprises, upon plant tissue culture, requiring that:
   (1) cells of a plant belonging to the genus Beta as cells to be cultured;
   (2) the cells are subjected to liquid culture;
   (3) the cells are cultured under aerobic conditions; and at the same time, meets 0 or at least one of the requirements 1. through 9. described below:
   1. auxin is added as a component in liquid medium;
   2. cytokinin is added as a component in liquid medium;
   3. the cells are cultured under light conditions;
   4. a part of the medium already provided for culture is added to a fresh liquid medium;
   5. ammonium ions ($NH_4^+$) are present in the medium in a far lower concentration than in LS medium;
   6. nitrate ions ($NO_3^-$) are present in the medium in a far lower concentration than in LS medium;
   7. potassium ions ($K^+$) are present in the medium in a far lower concentration than in LS medium;
   8. borate ions ($BO_3^{3-}$) are present in the medium in a far lower concentration than in LS medium; and,
   9. iron ions ($Fe^{2+}$) are present in the medium in a far lower concentration than in LS medium.

Fig. 1

# INTERNATIONAL SEARCH REPORT

International Application No  PCT/JP90/01142

**I. CLASSIFICATION OF SUBJECT MATTER** (if several classification symbols apply, indicate all) [6]

According to International Patent Classification (IPC) or to both National Classification and IPC

Int. Cl[5]    C12P19/60, 1/00//C12N5/04 (C12P19/60, C12R1:91)
              (C12P1/00, C12R1:91)

**II. FIELDS SEARCHED**

| Minimum Documentation Searched [7] | |
|---|---|
| Classification System | Classification Symbols |
| IPC | C12P1/00, 19/60, C12N5/04 |

Documentation Searched other than Minimum Documentation
to the Extent that such Documents are included in the Fields Searched [8]

Biological Abstracts Data Base (BIOSIS)
Chemical Abstracts Data Base (CA, REGISTRY)

**III. DOCUMENTS CONSIDERED TO BE RELEVANT [9]**

| Category [*] | Citation of Document, [11] with indication, where appropriate, of the relevant passages [12] | Relevant to Claim No. [13] |
|---|---|---|
| X | JP, A, 50-24494 (Kibun K.K.),<br>15 March 1975 (15. 03. 75),<br>(Family: none) | 1 |
| X | Journal of Food Science, Vol. 47, No. 1,<br>(1982), T. A. Weller et al.,<br>"Betalains in beet root tissue culture."<br>p. 162-3 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 30, 1990 (30. 11. 90) | December 17, 1990 (17. 12. 90) |
| International Searching Authority | Signature of Authorized Officer |
| Japanese Patent Office | |

Form PCT/ISA/210 (second sheet) (January 1985)